# EUROPEAN PATENT SPECIFICATION

(11) **EP 4 091 648 B1**
(45) Date of publication and mention of the grant of the patent: **02.10.2024**
(21) Application number: 22174019.4
(22) Date of filing: 18.05.2022
(51) Int. Cl.: A61J 1/16, B65D 25/10, A61M 5/00, B01L 9/06, A61J 1/06

(54) **TRANSPORT TRAY FOR PACKAGING UNITS**
TRANSPORTTABLETT FÜR VERPACKUNGSEINHEITEN
PLATEAU DE TRANSPORT POUR UNITÉS D'EMBALLAGE

(30) Priority: 18.05.2021 US 202163190086 P
(43) Date of publication of application: 23.11.2022
(73) Proprietor: Gerresheimer Glas GmbH, 40468 Düsseldorf (DE); STEVANATO GROUP S.P.A., 35017 Piombino Dese (PD) (IT)
(72) Inventor: HUTTERER, Nicole, 92442 Wackersdorf (DE); FRAAS, Andreas, 92224 Amberg (DE); COLCHADO, Ricardo, 76902 Corregidora, Queretaro (MX); FLYNN, Stephen, Peachtree City, GA, 30269 (US); MILLER, Braden, Havertown, PA, 19083 (US); ROSENMAN, Scott, Mount Laurel, NJ, 08054 (US); BONATI, Alessio, 35010 Cadoneghe (IT); GUASTI, Michele, 31100 Treviso (IT); CANESTRARO, Marco, 35010 Carmignano di Brenta (PADOVA) (IT); PRETE, Riccardo, 35020 Ponte San Nicolò (PD) (IT); BERTOLIN, Gianpaolo, 30033 Noale (IT)
(74) Representative: Fish & Richardson P.C.

(56) References cited:
- EP-A1- 3 345 587
- WO-A1-2011/135085
- US-A1- 2003 199 082
- US-A1- 2017 225 162
- US-A1- 2018 208 377
- US-A1- 2019 343 721

## Description

This disclosure relates to a tray for transporting packaging units.

In industrial contexts, products are generally transported and sold in packaging units. Packaging units can include vials, cartridges, ampoules, bottles, or pre-fillable syringes. In many industries, these different types of packaging units are collectively known as "primary packaging," i.e., the packaging that comes into direct contact with an end product. The end product may be a food product, a cosmetic product, or a pharmaceutical product. Primary packaging can undergo numerous manufacturing processes before being filled with the end product. During these processes, primary packaging is often transported and processed in batches.

US 10,703,539 B2 describes a carrier in which retaining protrusions provided at lower ends of apertures or receptacles of a supporting structure protrude inward in a radial direction into the apertures or receptacles for supporting vials in cooperation with transition regions or edge portions of the vials in such a manner that bottoms of the vials, or more generally the lower ends of the vials, jut out of the apertures or receptacles and are thus are freely accessible from the lower side of the carrier.

US 2018/0208377 A1 describes a supporting structure that has an upper side or base plane, which generally is formed as a plate and whose circumferential edge is formed flat. In the upper side, a plurality of openings is formed, which are arranged in rows and columns extending perpendicular to each other. The openings of adjacent rows or columns are arranged staggered relative to each other, which enables a higher packing density with the hexagonal arrangement of the peripheral webs. A plurality of axial connecting webs protrude perpendicularly from the underside of the supporting structure, which are connected to each other at their lower ends via circumferential bottom webs.

WO 2014/130349 A1 describes a tray with receptacles that are configured to support vials in different manners during different stages of processing. Each receptacle has a bottom opening defined at a bottom edge of lower portion of a cylindrical wall, and an inwardly extending lip surrounding each opening. The opening has an inner diameter slightly smaller than the outer diameter of the vial side wall, but slightly larger than the bottom wall, such that the inner edges of lip contact a portion of curved lower edge joining side wall and bottom wall. This configuration permits the vial to be supported by the tray while suspended, with a lower portion of the vial protruding downward from the bottom edge of the tray.

WO 2011/135085 A1, which accords with the preamble of claim 1, describes a packaging structure for containers for pharmaceutical use, comprising a tray having an open side for introducing and extracting a support plane of the containers, and a closing element of the open side of the tray, said support plane having a spatial prefixed distribution of seats in which said containers can be precisely positioned without mutual contact, whereby the structure comprises diversification means of the engaging configuration between said support plane and said tray in order to maintain unchanged the points inside the tray in which an end of the containers is placed, with the variation of the height of the containers associated from time to time to the support plane.

Aspects of the present disclosure aim to alleviate problems associated with known transport trays.

A transport tray according to the present invention is set out in claim 1. Further advantageous developments of the present invention are set out in the dependent claims. According to the present invention, a transport tray includes a plurality of sleeves that are each configured to receive a substantially cylindrical container having a cylindrical container wall and a container bottom surface arranged orthogonally to the container wall. Each sleeve includes a top opening, a bottom opening, a sleeve wall that extends along a sleeve axis between the top opening and the bottom opening and is configured to abut at least a portion of the container wall of a respective container, and one or more support feet that are adjacent to the bottom opening and extend from the sleeve wall towards the sleeve axis, wherein each support foot comprises a bottom surface and a top surface configured to abut the container bottom surface of a respective container, such that a gap is formed between the support foot bottom surface and the container bottom surface along the sleeve axis. The one or more support feet comprise a thermally conductive polymer;wherein the transport tray further comprises a plate portion connected to the top opening of each sleeve and comprising a plurality of comer regions, and a raised bumper arranged at least at each corner region of the plate portion, wherein the raised bumper has a height that is greater than a thickness of the plate portion; and wherein a perimeter of the plate portion comprises the raised bumpers and an edge portion having the same thickness as the plate portion arranged between each pair of adjacent raised bumpers.

In some instances, each sleeve can include a ring that connects the one or more support feet to the bottom opening and that comprises a thermally conductive polymer. Further, each sleeve can be a monolithic structure that comprises a thermally conductive polymer.

In addition to the respective sleeves, the entire transport tray may be a monolithic structure that comprises a thermally conductive polymer. For example, the transport tray may be manufactured by injection molding a thermally conductive polymer.

The thermally conductive polymer may comprise an aluminum filler, a zinc filler, or a combination thereof.

Each sleeve wall may include a closed cylinder that encircles the sleeve axis.

The top opening of each sleeve may be larger than the bottom opening, such that the sleeve wall forms an angle greater than 0° relative to the sleeve axis.

According to the present invention, the transport tray includes a plate portion connected to the top opening of each sleeve. Preferably, a connecting portion may connect the sleeve walls of at least two adjacent sleeves to the plate portion. According to the present invention, the plate portion comprises a plurality of corner regions and a raised bumper arranged at each corner region. Such a raised bumper has a height that is greater than a thickness of the plate portion. Preferably, each raised bumper may include at least one projection that extends outward from a perimeter of the plate portion. According to the present invention, the perimeter of the plate portion includes the raised bumpers and an edge portion having the same thickness as the plate portion arranged between each pair of adjacent raised bumpers.

These and other embodiments described herein may provide one or more of the following benefits. The transport tray may be suitable for transferring heat to and from the packaging units. For example, the transport tray may be used in a lyophilization process. At the same time, the transport tray may securely support the packaging units in other processes, such as direct filling processes. Overall, the transport tray may reduce the need to handle individual packaging units during the manufacturing process. Further, the design of the transport tray may be compatible with existing equipment. The transport tray may also prove to be robust.

Certain embodiments will now be described, by way of example only, with reference to the accompanying drawings, in which:
Figure 1 shows a transport tray according to the present disclosure from above;
Figure 2 shows a perspective view of the transport tray of Figure 1;
Figure 3 shows a partial perspective view of a bottom of the transport tray of Figure 1;
Figures 4A and 4B show cross-sectional views through the transport tray of Figures 1 to 3;
Figures 5A and 5B schematically show multiple transport trays placed next to one another on a table; and
Figure 5C shows a partial perspective view of the transport tray of Figure 2.

Like reference numbers and designations in the various drawings indicate like elements.

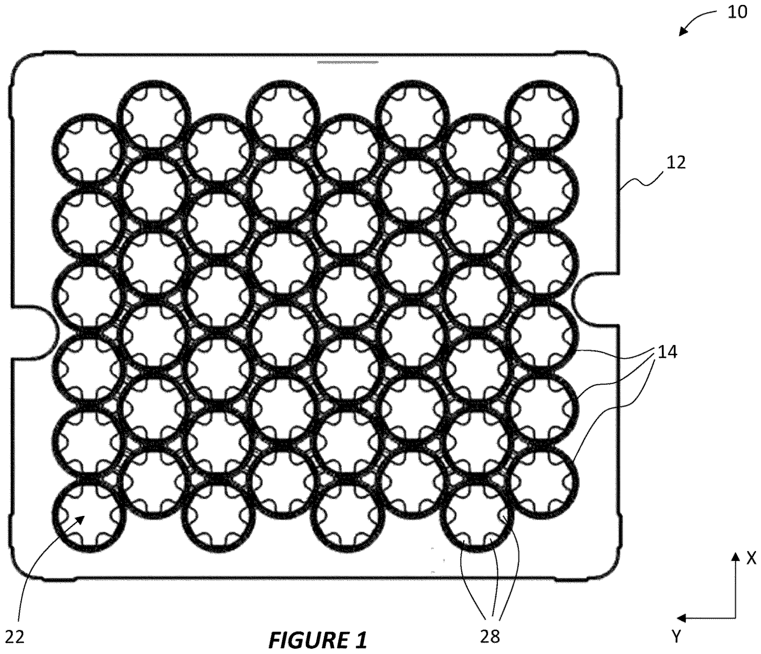

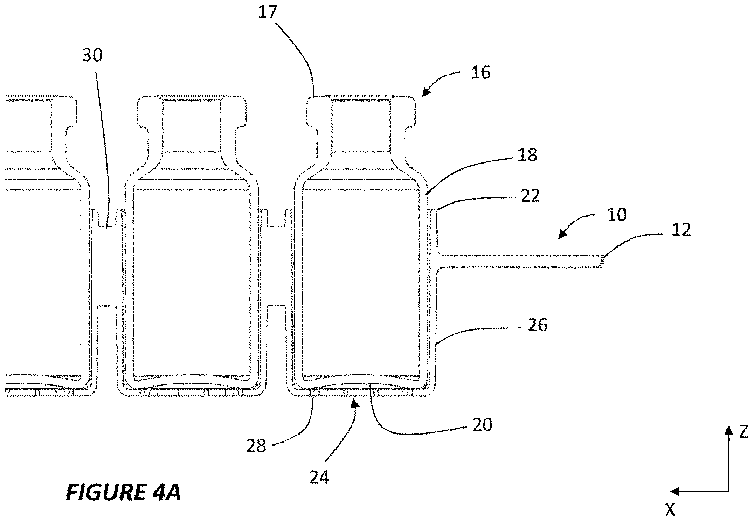

Figure 1 shows a transport tray 10 according to the present disclosure from above. The transport tray 10 includes a plate portion 12 and a plurality of sleeves 14. The sleeves 14 are each configured to receive a substantially cylindrical container 16 having a cylindrical container wall 18 and a container bottom surface 20 arranged orthogonally to the container wall 18 (Figure 4A). The containers 16 can serve as primary packaging for various types of end products. Although the expression "primary packaging" can encompass vials, cartridges, ampoules, bottles, and syringes to name a few examples, the figures of the present disclosure illustrate vials. The transport tray 10 can be used to group and transport the vials 16 throughout different manufacturing processes, for example.

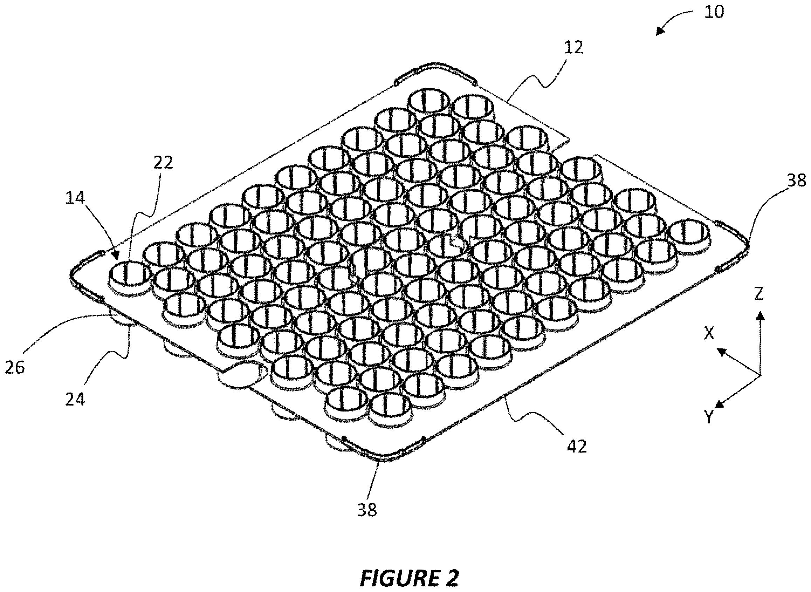

As shown in Figure 2, each sleeve 14 includes a top opening 22, a bottom opening 24, a sleeve wall 26 that extends along a sleeve axis between the top opening 22 and the bottom opening 24. Referring to the coordinate axes shown in Figure 2, the sleeve axis of each sleeve 14 extends along the Z-axis.

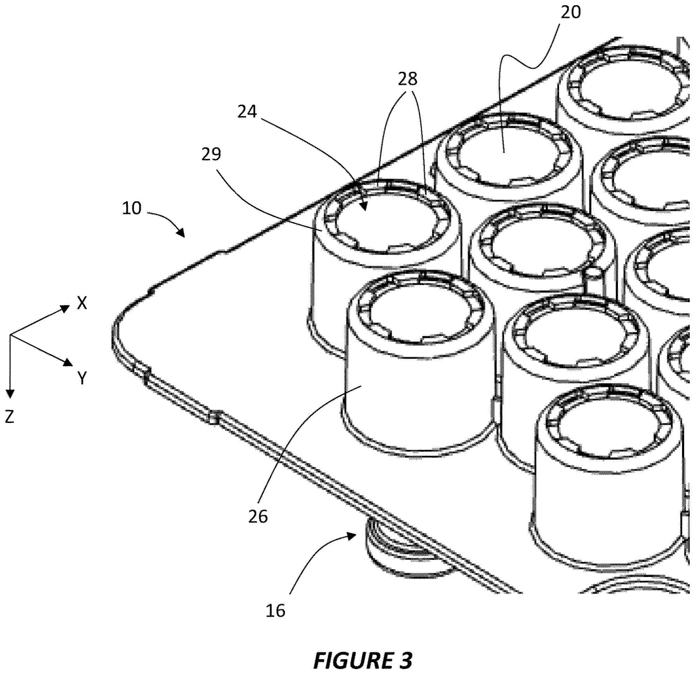

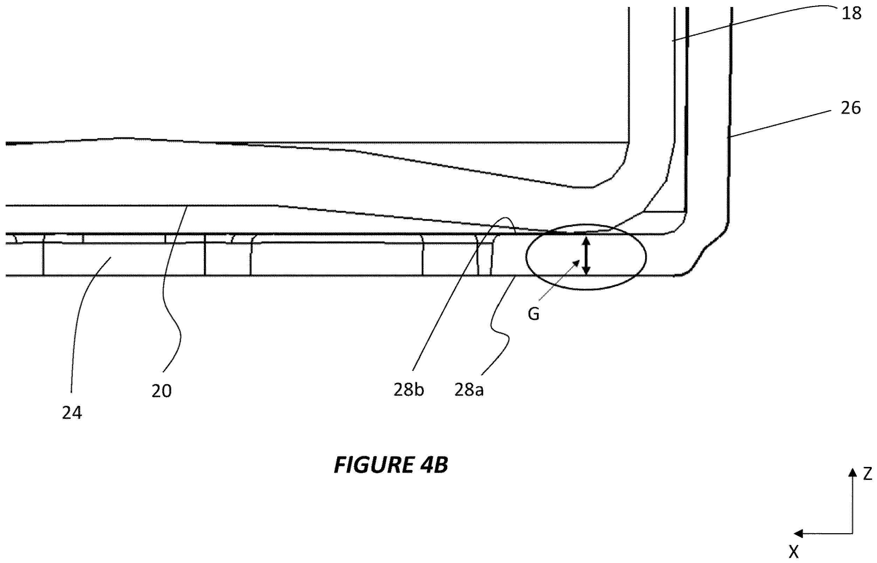

Figures 3 and 4 show the transport tray 10 of Figures 1 and 2 with a plurality of containers 16 arranged in the sleeves 14. The containers 16 are arranged in the sleeves 14 such that the sleeve wall 26 faces the container wall 18 (Figure 4A). The container 16 includes an opening 17 that is arranged facing the top opening 22 of the sleeve 14, while the bottom surface 20 of the container is adj acent to the bottom opening 24 of the sleeve 14. Each sleeve 14 includes one or more support feet 28 that are adjacent to the bottom opening 24 and extend from the sleeve wall 26 towards the sleeve axis. Each support foot 28 comprises a bottom surface 28a and a top surface 28b configured to abut the container bottom surface 20 of the container 16 received in the sleeve. The support foot 28 is designed so that a gap G is formed between the support foot bottom surface 28a and the container bottom surface 20 along the sleeve axis (Figure 4B).

Accordingly, the transport tray 10 rests on the collective support foot bottom surfaces 28a of each of the support feet 28, while each container bottom surface 20 rests entirely on the support foot top surfaces 28b of the support feet 28 in a respective sleeve 14. As shown in Figures 4A and 4B, the container bottom surface 20 does not extend past the support feet 28. In other words, the container bottom surface 20 is separated from the surface on which the transport tray 10 rests. In practice, this may mean that the support foot top surface 28b makes contact with the lowest point of the container bottom surface 20.

According to the present disclosure, the one or more support feet 28 include a thermally conductive polymer. For example, the one or more support feet 28 are formed as a single part, e.g., of injection molded plastic with thermally conductive fillers. Such support feet 28 may be attached to the sleeves 14 using insert molding techniques. For example, the filler may include an aluminum filler, a zinc filler, or a combination thereof. In some instances, the one or more support feet 28 can be attached to the bottom opening 24 by a ring 29 (Figure 3). The ring 29 may also include a thermally conductive polymer. The ring 29 and the one or more support feet 28 may be integrally formed, i.e., form a monolithic structure. In some instances, the entire sleeve 14, or even the entire support tray 10, may be a monolithic structure. For example, the transport tray 10 may be manufactured by injection molding a thermally conductive polymer. As described above, the container bottom surface 20 rests on the support foot top surfaces 28b of each sleeve 14.

Since at least the one or more support feet 28 include thermally conductive polymer, the support feet 28 are able to transfer heat to and from the container bottom surface 20. Accordingly, the transport tray 10 may be used during a lyophilization process with the containers 16 remaining in the sleeves 14. Since the container bottom surface 20 does not protrude through the bottom opening 24 of the sleeve 14, the same transport tray 10 can be used for other processes, e.g., a direct fill process. Thus, the transport tray 10 according to the present disclosure may contribute to the efficiency of the manufacturing process for primary packaging.

The transport tray 10 may include any number of the following features that may improve the robustness and heat transfer of the transport tray 10.

As shown in Figure 3, for example, each sleeve wall 26 may include a closed cylinder that completely encircles the sleeve axis and, thus, a portion of the container wall 18. In cases in which the entire sleeve 14 includes a thermally conductive polymer, this closed cylinder design can increase the amount of thermally conductive polymer that surrounds the container wall 18 and improve heat transfer, e.g., during the lyophilization process. At the same time, the closed cylinder may improve the robustness of the sleeve 14 and transport tray 10, respectively.

The top opening 22 of each sleeve 14 may be larger than the bottom opening 24, such that the sleeve wall forms an angle greater than 0° relative to the sleeve axis. Such a taper may make it easier to retrieve the container 16 from the sleeve 14 despite the closed cylinder design described above.

As shown in Figure 4A, the transport tray 10 may include a connecting portion 30 that connects the sleeve walls 26 of at least two adjacent sleeves to the plate portion 12. The connecting portion 30 can also increase the volume of thermally conductive polymer and, thus, the heat transfer through the transport tray 10 and its overall robustness.

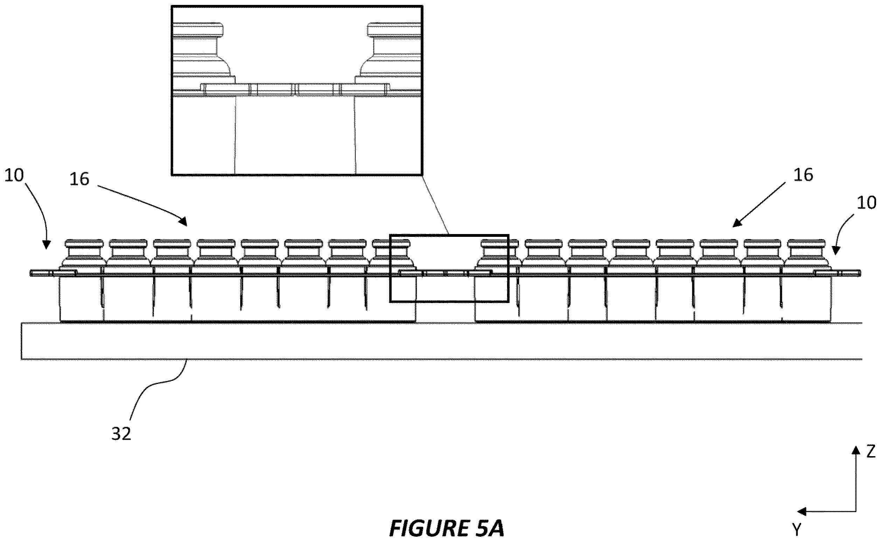

Figures 5A to 5C show optional features of the transport tray 10 that may enable several transport trays 10 to be neatly stacked next to one another. As shown in Figure 5A, a first and second transport tray 10 are each loaded with a plurality of containers 16 and placed next to one another on a table 32. For example, the table 32 may be used for a lyophilization process to rapidly cool the containers 16 and their contents.

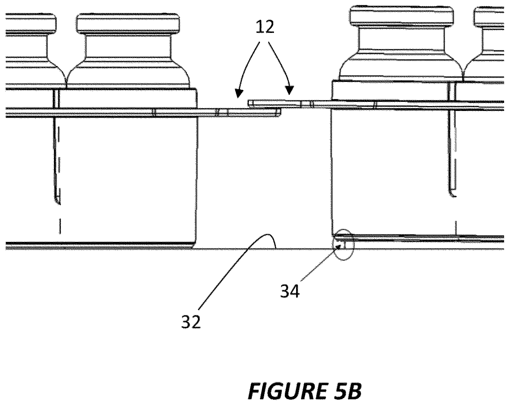

As shown in the enlarged portion of Figure 5A, the plate portions 12 of each transport tray 10 are arranged closely next to each other without touching. Such an arrangement may allow the greatest possible number of containers 16 to be placed on the table at once. Figure 5B shows a scenario in which adjacent transport trays 10 have been placed so closely together that the plate portions 12 overlap. This overlap in the plate portions creates a gap 34 between the table 32 and the transport tray 10 on the right. This gap 34 may reduce the efficiency of the heat transfer between the table 32, the transport tray 10, and the containers 16.

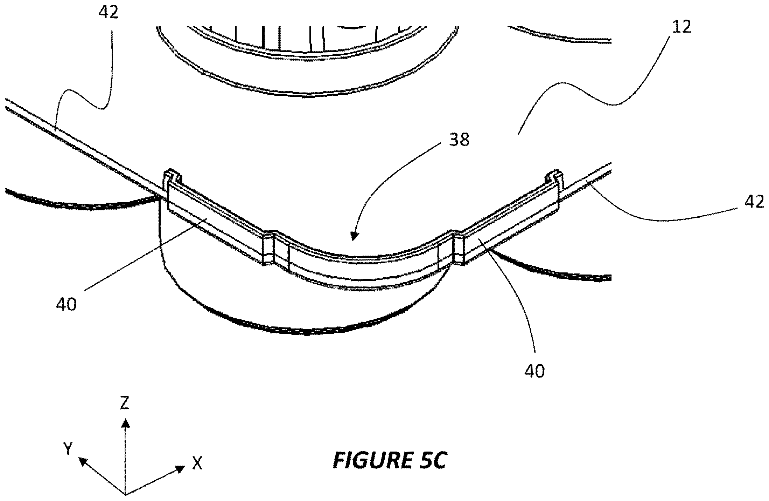

In order to prevent the overlap shown in Figure 5B, each corner region 36 of the plate portion 12 may be provided with one or more features that reduce the likelihood that adjacent transport trays 10 overlap one another. As shown in Figure 5C, each corner region 36 of the plate portion 12 includes a raised bumper 38. In the context of this disclosure, "raised" can mean that a height of the bumper 38 along the Z-axis is greater than a thickness of the plate portion 12 along the Z-axis. When two transport trays 10 are brought close together, the matching bumpers 38 may come into contact and prevent the plate portions 12 from overlapping.

In some instances, the raised bumper 38 can include a first and a second projection 40 that increase the stability of the raised bumper 38. In Figure 5C, the first projection 40 extends outward from the plate portion 12 along the X-axis, and the second projection 40 extends outward from the plate portion 12 along the Y-axis.

The raised bumper 38 can include a strip of material that continuously wraps around the corner region 36 of the plate portion. Each end of the raised bumper 38 can abut an edge portion 42 of the plate portion 12 that does not have an increased thickness (see also Figure 2). The combination of alternating raised bumpers 38 and edge portions 42 improves air flow during injection molding of the transport tray 10.

A number of embodiments have been described. Nevertheless, numerous alternative embodiments within the scope of the claims will be readily appreciated by those skilled in the art. The presently described embodiments are not to be taken as limiting the scope of the invention that is defined by the claims.

## Claims

1. A transport tray (10) comprises
a plurality of sleeves (14), wherein each sleeve (14) is configured to receive a substantially cylindrical container (16) having a cylindrical container wall (18) and a container bottom surface (20) arranged orthogonally to the container wall (18);
wherein each sleeve (14) comprises
a top opening (22),
a bottom opening (24),
a sleeve wall (26) that extends along a sleeve axis between the top opening (22) and the bottom opening (24) and is configured to abut at least a portion of the container wall (18) of a respective container (16), and
one or more support feet (28) that are adjacent to the bottom opening (24) and extend from the sleeve wall (26) towards the sleeve axis, wherein each support foot (28) comprises a bottom surface (28a) and a top surface (28b) configured to abut the container bottom surface (20) of a respective container (16), such that a gap (G) is formed between the support foot bottom surface (28a) and the container bottom surface (20) along the sleeve axis, **characterized in that** the one or more support feet (28) comprise a thermally conductive polymer;
wherein the transport tray (10) further comprises
a plate portion (12) connected to the top opening (22) of each sleeve (14) and comprising a plurality of corner regions, and
a raised bumper (38) arranged at least at each corner region of the plate portion (12), wherein the raised bumper (38) has a height that is greater than a thickness of the plate portion (12); and
wherein a perimeter of the plate portion (12) comprises the raised bumpers (38) and an edge portion (42) having the same thickness as the plate portion (12) arranged between each pair of adjacent raised bumpers (38).

2. The transport tray (10) according to claim 1, wherein each sleeve (14) further comprises a ring (29) that connects the one or more support feet (28) to the bottom opening (24), wherein the ring (29) comprises a thermally conductive polymer.

3. The transport tray (10) according to claim 2, wherein each sleeve (14) is a monolithic structure that comprises a thermally conductive polymer.

4. The transport tray (10) according to claim 3, wherein the transport tray (10) is a monolithic structure that comprises a thermally conductive polymer.

5. The transport tray (10) according to claim 4, wherein the transport tray (10) is manufactured by injection molding a thermally conductive polymer.

6. The transport tray (10) according to any one of claims 1 to 5, wherein the thermally conductive polymer comprises an aluminum filler.

7. The transport tray (10) according to any one of the preceding claims, wherein the thermally conductive polymer comprises a zinc filler.

8. The transport tray (10) according to any one of the preceding claims, wherein each sleeve wall (26) comprises a closed cylinder that encircles the sleeve axis.

9. The transport tray (10) according to any one of the preceding claims, wherein the top opening (22) of each sleeve (14) is larger than the bottom opening (24), such that the sleeve wall (26) forms an angle greater than 0° relative to the sleeve axis.

10. The transport tray (10) according to any one of the preceding claims, further comprising:
a connecting portion (30) that connects the sleeve walls (26) of at least two adjacent sleeves (14) to the plate portion (12).

11. The transport tray (10) according to claim 1, wherein each raised bumper (38) comprises at least one projection (40) that extends outward from the perimeter of the plate portion (12).

## Patentansprüche

1. Transporttablett (10), umfassend
eine Vielzahl von Hülsen (14), wobei jede Hülse (14) zur Aufnahme eines im Wesentlichen zylindrischen Behälters (16) ausgelegt ist, der eine zylindrische Behälterwand (18) und eine orthogonal zu der Behälterwand (18) angeordnete Behälterbodenfläche (20) aufweist,
wobei jede Hülse (14) Folgendes umfasst:
eine obere Öffnung (22),
eine untere Öffnung (24),
eine Hülsenwand (26), die sich entlang einer Hülsenachse zwischen der oberen Öffnung (22) und der unteren Öffnung (24) erstreckt und dazu ausgelegt ist, an mindestens einen Abschnitt der Behälterwand (18) eines jeweiligen Behälters (16) anzuliegen, und
einen oder mehrere Stützfüße (28), die der unteren Öffnung (24) benachbart sind und sich von der Hülsenwand (26) zu der Hülsenachse hin erstrecken, wobei jeder Stützfuß (28) eine untere Fläche (28a) und eine obere Fläche (28b) umfasst, die dazu ausgelegt sind, an der Behälterbodenfläche (20) eines jeweiligen Behälters (16) anzuliegen, so dass zwischen der unteren Fläche (28a) des Stützfußes und der Behälterbodenfläche (20) entlang der Hülsenachse ein Spalt (G) ausgebildet ist, **dadurch gekennzeichnet, dass** der eine oder die mehreren Stützfüße (28) ein wärmeleitendes Polymer umfassen,
wobei das Transporttablett (10) ferner Folgendes umfasst: einen Plattenabschnitt (12), der mit der oberen Öffnung (22) jeder Hülse (14) verbunden ist und eine Vielzahl von Eckbereichen umfasst, und
eine erhabene Stoßleiste (38), die mindestens an jedem Eckbereich des Plattenabschnitts (12) angeordnet ist, wobei die erhabene Stoßleiste (38) eine Höhe hat, die größer als eine Dicke des Plattenabschnitts (12) ist, und wobei ein Umfang des Plattenabschnitts (12) die erhaben Stoßleisten (38) und einen zwischen jedem Paar benachbarter erhabener Stoßleisten (38) angeordneten Randabschnitt (42) mit derselben Dicke wie der Plattenabschnitt (12) umfasst.

2. Transporttablett (10) nach Anspruch 1, wobei jede Hülse (14) ferner einen Ring (29) umfasst, der den einen oder die mehreren Stützfüße (28) mit der unteren Öffnung (24) verbindet, wobei der Ring (29) ein wärmeleitendes Polymer umfasst.

3. Transporttablett (10) nach Anspruch 2, wobei jede Hülse (14) eine monolithische Struktur ist, die ein wärmeleitendes Polymer umfasst.

4. Transporttablett (10) nach Anspruch 3, wobei das Transporttablett (10) eine monolithische Struktur ist, die ein wärmeleitendes Polymer umfasst.

5. Transporttablett (10) nach Anspruch 4, wobei das Transporttablett (10) durch Spritzgießen eines wärmeleitenden Polymers hergestellt ist.

6. Transporttablett (10) nach einem der Ansprüche 1 bis 5, wobei das wärmeleitende Polymer einen Aluminiumfüllstoff umfasst.

7. Transporttablett (10) nach einem der vorhergehenden Ansprüche, wobei das wärmeleitende Polymer einen Zinkfüllstoff umfasst.

8. Transporttablett (10) nach einem der vorhergehenden Ansprüche, wobei jede Hülsenwand (26) einen geschlossenen Zylinder umfasst, der die Hülsenachse umschließt.

9. Transporttablett (10) nach einem der vorhergehenden Ansprüche, wobei die obere Öffnung (22) jeder Hülse (14) größer als die untere Öffnung (24) ist, so dass die Hülsenwand (26) bezüglich der Hülsenachse einen Winkel von größer 0° bildet.

10. Transporttablett (10) nach einem der vorhergehenden Ansprüche, ferner umfassend:
einen Verbindungsabschnitt (30), der die Hülsenwände (26) mindestens zweiter benachbarter Hülsen (14) mit dem Plattenabschnitt (12) verbindet.

11. Transporttablett (10) nach Anspruch 1, wobei jede erhabene Stoßleiste (38) mindestens einen Vorsprung (40) umfasst, der sich von dem Umfang des Plattenabschnitts (12) nach außen erstreckt.

## Revendications

1. Plateau de transport (10) comprenant
une pluralité de manchons (14), chaque manchon (14) étant conçu pour recevoir un récipient (16) sensiblement cylindrique ayant une paroi de récipient (18) cylindrique et une surface de fond de récipient (20) disposée orthogonalement à la paroi de récipient (18) ;
chaque manchon (14) comprenant
une ouverture supérieure (22),
une ouverture inférieure (24),
une paroi de manchon (26) qui s'étend le long d'un axe de manchon entre l'ouverture supérieure (22) et l'ouverture inférieure (24) et est conçue pour entrer en butée contre au moins une partie de la paroi de récipient (18) d'un récipient (16) respectif, et
un ou plusieurs pieds de support (28) qui sont adjacents à l'ouverture inférieure (24) et s'étendent depuis la paroi de manchon (26) vers l'axe de manchon, chaque pied de support (28) comprenant une surface inférieure (28a) et une surface supérieure (28b) conçue pour entrer en butée contre la surface de fond de récipient (20) d'un récipient (16) respectif, de sorte qu'un espace (G) est formé entre la surface inférieure (28a) de pied de support et la surface de fond de récipient (20) le long de l'axe de manchon, **caractérisé en ce que** le ou les pieds de support (28) comprennent un polymère thermiquement conducteur ;
le plateau de transport (10) comprenant en outre une partie formant plaque (12) raccordée à l'ouverture supérieure (22) de chaque manchon (14) et comprenant une pluralité de régions de coin, et
un élément de butée surélevé (38) disposé au moins au niveau de chaque région de coin de la partie formant plaque (12), l'élément de butée surélevé (38) ayant une hauteur supérieure à une épaisseur de la partie formant plaque (12); et
un périmètre de la partie formant plaque (12) comprenant les éléments de butée surélevés (38) et une partie de bord (42) ayant la même épaisseur que la partie formant plaque (12) disposée entre chaque paire d'éléments de butée surélevés (38) adjacents.

2. Plateau de transport (10) selon la revendication 1, chaque manchon (14) comprenant en outre une bague (29) qui raccorde le ou les pieds de support (28) à l'ouverture inférieure (24), la bague (29) comprenant un polymère thermiquement conducteur.

3. Plateau de transport (10) selon la revendication 2, chaque manchon (14) étant une structure monolithique qui comprend un polymère thermiquement conducteur.

4. Plateau de transport (10) selon la revendication 3, le plateau de transport (10) étant une structure monolithique qui comprend un polymère thermiquement conducteur.

5. Plateau de transport (10) selon la revendication 4, le plateau de transport (10) étant fabriqué par moulage par injection d'un polymère thermiquement conducteur.

6. Plateau de transport (10) selon l'une quelconque des revendications 1 à 5, le polymère thermiquement conducteur comprenant une charge d'aluminium.

7. Plateau de transport (10) selon l'une quelconque des revendications précédentes, le polymère thermiquement conducteur comprenant une charge de zinc.

8. Plateau de transport (10) selon l'une quelconque des revendications précédentes, chaque paroi de manchon (26) comprenant un cylindre fermé qui encercle l'axe de manchon.

9. Plateau de transport (10) selon l'une quelconque des revendications précédentes, l'ouverture supérieure (22) de chaque manchon (14) étant plus grande que l'ouverture inférieure (24), de sorte que la paroi de manchon (26) forme un angle supérieur à 0° par rapport à l'axe de manchon.

10. Plateau de transport (10) selon l'une quelconque des revendications précédentes, comprenant en outre :
une partie de raccordement (30) qui raccorde les parois de manchon (26) d'au moins deux manchons (14) adjacents à la partie formant plaque (12).

11. Plateau de transport (10) selon la revendication 1, chaque élément de butée surélevé (38) comprenant au moins une saillie (40) qui s'étend vers l'extérieur depuis le périmètre de la partie formant plaque (12).
